# EUROPÄISCHE PATENTANMELDUNG

(11) **EP 1 093 764 A1**
(43) Veröffentlichungstag der Anmeldung: **25.04.2001**
(21) Anmeldenummer: 00122636.4
(22) Anmeldetag: 17.10.2000
(51) Int. Cl.: A61B 19/08, A61B 19/00

(54) **Fixiervorrichtung für ein, im Sterilbereich bei Operationen einsetzbares Bedienelement**

(30) Priorität: 19.10.1999 DE 19950440
(71) Anmelder: Lemke, Rosemarie, 82194 Gröbenzell (DE); Lemke, Norbert, 82194 Gröbenzell (DE)
(72) Erfinder: Lemke, Rosemarie, 82194 Gröbenzell (DE); Lemke, Norbert, 82194 Gröbenzell (DE)
(74) Vertreter: Rösler, Uwe, Dipl.-Phys.

(57) **Zusammenfassung**

Beschrieben wird eine Fixiervorrichtung für wenigstens ein, im Sterilbereich bei Operationen einsetzbares Bedienelement oder Fixierelement, das räumlich weitgehend frei positionierbar und fixierbar gelagert ist, wobei der Sterilbereich vom Nicht-Sterilbereich im Wesentlichen durch ein tuchartiges Abdeckmittel abgegrenzt ist.

Die Erfindung zeichnet sich dadurch aus, dass ein, im Nicht-Sterilbereich befindliches Haltemittel vorgesehen ist, das wenigstens getrennt durch das Abdeckmittel über eine Wirkfläche mit dem Bedien- oder Fixierelement in Wirkverbindung tritt, und dass die Wirkverbindung auf magnetischen Kräften beruht.

## Beschreibung

### Technisches Gebiet

Die Erfindung bezieht sich auf eine Fixiervorrichtung für wenigstens ein, im Sterilbereich bei Operationen einsetzbares Bedienelement oder Fixierelement, das räumlich weitgehend frei positionierbar und fixierbar gelagert ist, wobei der Sterilbereich vom Nicht-Sterilbereich im Wesentlichen durch ein tuchartiges Abdeckmittel abgegrenzt ist.

### Stand der Technik

Die Durchführung endoskopischer Operationen, vorzugsweise am menschlichen Körper, erfordert von den, an der Operation beteiligten Ärzten einen präzisen Umgang mit den, durch Körperöffnungen intrakorporal eingebrachten endoskopischen Operationsbestecke. Insbesondere bei der Vornahme von Operationsmaßnahmen durch die Bauchdecke hindurch bedarf es mehrerer unabhängig voneinander zu bedienender endoskopischer Instrumente, die jeweils von unterschiedlichen, an der Operation beteiligten Ärzten handzuhaben sind. Für die optische Überwachung der Operation wird ein, in aller Regel mit einer Videokamera verbundenes Sichtendoskop in das Körperinnere eingeführt und je nach Operationsbereich an diesem ausgerichtet und entsprechend positioniert. Eine derartige optische Überwachung der im Körper vorzunehmenden operativen Eingriffe erfordert jedoch eine ständige Nachführung des distalen Endes der endoskopisch ausgebildeten Bildaufnahmeoptik, sodass diese Instrumente während der gesamten Dauer des operativen Eingriffes kontrolliert nachgeführt werden müssen. Die zum Teil zeitlich sehr lang andauernden operativen Eingriffe führen zwangsläufig bei den, an der Operation beteiligten Ärzten zu Ermüdungserscheinungen, insbesondere bei jenen, die lediglich mit der Positionierung und Nachführung der jeweiligen Instrumente befasst sind, zumal dies monotone Tätigkeiten sind, die jedoch ebenso ein Höchstmaß an Konzentration erfordern.

Zur Entlastung des Operationspersonals sind stativartige Anordnungen bekannt, die ein Positionieren endoskopischer Instrumente relativ zu einem Operationsbereich ermöglichen. Hierzu zählen am Boden stehende oder am Operationstisch zu befestigende Stativanordnungen, die über eine Vielzahl, jeweils mit Gelenken verbundener Stativarme verfügen, wobei die einzelnen Stativgelenke durch Stellschrauben oder mittels rotatorisch bedienbare Fixiermechanismen festgestellt werden können. Neben den manuell bedienbaren Stativanordnungen sind auch elektromotorisch angetriebene Stativanordnungen bekannt, die über ein entsprechendes Steuergerät dreidimensional positioniert werden können. Allen bekannten Stativanordnungen liegt das gemeinsame Funktionsziel zu Grunde, ein für die Durchführung bzw. Überwachung erforderliches Operationsinstrument nahe dem intrakorporalen Arbeitsbereich, in dem der Arzt seinen operativen Eingriff vornimmt, zu positionieren. Hierzu ist das entsprechende operative Instrument am distalen Endbereich der Stativanordnung angebracht, das es gilt einerseits räumlich möglichst ungehindert zu positionieren und andererseits in einer vorgegebenen räumlichen Position zu arretieren sowie die Arretierung auch wieder leicht zu lösen.

Neben den unterschiedlichen Ausgestaltungsformen bekannter Stativanordnungen, die sich zwar technisch durch unterschiedliche konstruktive Befestigungs- bzw. Arretierungsmechanismen unterscheiden, doch sich allesamt durch aufwändige Konstruktionen auszeichnen, spielen insbesondere die Anschaffungskosten bei der Wahl für oder gegen ein bestimmtes Handhabungssystem eine wichtige Rolle.

Ein weiterer sehr wichtiger Aspekt bei der konstruktiven Auslegung vorstehend bezeichneter Stativ- bzw. Fixiervorrichtungen ist die Möglichkeit ihrer Sterilisierbarkeit, zumal sie für den Einsatz im Sterilbereich von Operationsräumen vorgesehen sind. Klemm- oder Gelenkverbindungen sind auf Grund ihres mehrteiligen Aufbaus auf Grund einer Vielzahl von Spalten und Zwischenräumen umständlich bzw. aufwändig zu sterilisieren. Häufig werden die Stativ- bzw. Fixierkomponenten mit so genannten sterilen Einweghüllen umgeben, durch die jedoch die Bedienbarkeit der einzelnen Stativkomponenten erheblich erschwert wird. Auch stellt die Notwendigkeit der Verwendung der sterilen Umhüllungen einen nicht zu vernachlässigenden zusätzlichen Kostenfaktor dar, zumal für jede einzelne Operation wenigsten eine Umhüllung verbraucht wird.

### Darstellung der Erfindung

Der Erfindung liegt die Aufgabe zu Grunde eine Fixiervorrichtung für wenigstens ein, im Sterilbereich bei Operationen einsetzbares Bedienelement oder Fixierelement, das räumlich weitgehend frei positionierbar und fixierbar gelagert ist, derart weiterzubilden, dass der arbeitstechnische und investitionsbedingte Aufwand für die Sterilisation der im Sterilbereich einzusetzenden Fixiervorrichtung auf ein Minimum reduziert werden soll. Kostenintensive Verbrauchsmaterialien, wie sie bei bekannten Stativ- bzw. Fixiervorrichtungen notwendig sind, sollen vollständig vermieden werden. Überdies soll die Fixiervorrichtung eine leichte Bedienbarkeit für den Operateur ermöglichen, insbesondere soll der konstruktionsbedingte Aufwand für eine freie räumliche Positionierbarkeit der stativartig ausgebildeten Fixiervorrichtung klein und einfach gehalten sein. Ein wesentlicher Aspekt der Erfindung betrifft überdies eine möglichst kostengünstige Lösung anzubieten, die vor allem im täglichen Einsatz keine oder nur geringe Betriebs- und Wartungskosten aufwirft.

Die Lösung der der Erfindung zu Grunde liegende Aufgabe ist im Anspruch 1 angegeben. Den Erfindungsgedanken vorteilhaft weiterbildende Merkmale sind Gegenstand der Unteransprüche sowie der Beschreibungseinleitung und den Zeichnungen zu entnehmen.

Die der Erfindung zu Grunde liegende Idee berücksichtigt das bei allen Operationen eingesetzte Operationsabdecktuch, das den nichtsterilen Bereich vom Sterilbereich abtrennt.

Die zumeist aus Stoff gefertigten Operationsabdecktücher (die weiteren Ausführungen gelten grundsätzlich auch für Abdecktücher aus anderen Materialien bzw. Materialzusammensetzungen, beispielsweise beschichtetes Papier, Kunststoffabdeckfolien etc.) sind zumeist aus grün eingefärbter Baumwolle oder Textilstoffen gefertigt und als solche durch entsprechende Waschvorgänge sterilisierbar und wieder verwendbar. Diesbezügliche tuchartige Abdeckmittel werden bei Operationen über den zu behandelnden Patienten gelegt und nur am Körperbereich, an dem der operative Eingriff erfolgt, verbleibt eine entsprechende Aussparung. Alle Instrumente und Gegenstände, die sich oberhalb des tuchartigen Abdeckmittels befinden und somit dem Operateur zugewandt sind, müssen steril sein, wohingegen Gegenstände, die sich unter des tuchartigen Abdeckmittels befinden, keinerlei oder nur stark geminderte Sterilitätsanforderungen entsprechen müssen. Dies macht sich die Erfindung zu Nutze.

Erfindungsgemäß ist eine Fixiervorrichtung für wenigstens ein im Sterilbereich bei Operationen einsetzbares Bedienelement oder Fixierelement, das räumlich weitgehend frei positionierbar und fixierbar gelagert ist, wobei der Sterilbereich vom Nichtsterilbereich im Wesentlichen durch ein tuchartiges Abdeckmittel abgegrenzt ist, dadurch weitergebildet, dass ein, im Nicht-Sterilbereich befindliches Haltemittel vorgesehen ist, das wenigstens getrennt durch das Abdeckmittel über eine Wirkfläche mit dem Bedien- oder Fixierelement in Wirkverbindung tritt, wobei die Wirkverbindung auf magnetischen Kräften beruht.

Die erfindungsgemäße Fixiervorrichtung setzt sich somit grundsätzlich aus zwei Bauteilgruppen zusammen, nämlich jene die unsteril unterhalb des Abdeckmittels angeordnet sind, und jene, die sich im Sterilbereich oberhalb des Abdeckmittels befinden. Die unsterilen Komponenten der Fixiervorrichtung umfassen vorzugsweise einen Befestigungsfuß, der entweder als ein, auf dem Boden stehendes Standbein oder als Klemmvorrichtung zum lösbar festen Fixieren an einer, den Operationstisch umlaufenden Befestigungsschiene ausgebildet ist.

Vorzugsweise um eine Vertikalachse, stufenlos höhenverstellbar ist das Haltemittel mit dem Standbein oder der Klemmvorrichtung verbunden und bildet mit diesem/dieser eine Einheit. Das Haltemittel selbst ist in seiner einfachsten Ausführungsform als Permanentmagnet ausgebildet, der eine Ebene, pfannenartig oder konkavsphärisch gekrümmte Wirkfläche aufweist. Überdies ist es optional neben der stufenlos vertikalen Höhenverstellbarkeit des Haltemittels möglich, das Haltemittel derart relativ zu einer horizontal angeordneten Ebene zu neigen, sodass die Wirkfläche des Haltemittels eine geneigte Stellung relativ zur Horizontalen, vorzugsweise um 45° geneigt, einnimmt.

Alternativ zur Verwendung eines Permanentmagneten als Haltemittel eignet sich auch ein Elektromagnet, dessen Magnetkräfte in Abhängigkeit des, den Elektromagneten speisenden Stromes bestimmt sind; hierdurch kann der Magnet wenigstens zwischen einer Ein- und Ausstellung geschaltet werden, überdies kann der E-Magnet vorzugsweise zwischen der Ein- und Ausstellungen kontinuierlich geregelt werden, das heißt, die magnetische Feldstärke lässt sich stufenlos auf einen maximalen Feldstärkewert regeln.

Die nichtsterilen Komponenten der Fixiervorrichtung werden vorzugsweise in unmittelbarer Nähe zum Operationstisch angeordnet, sodass das, den zu Behandelnden, auf dem Operationstisch liegenden Patienten, steril abdeckende Operationstuch zugleich auch das Haltemittel mit den entsprechenden Befestigungsvorkehrungen überdeckt. Das Operationstuch, das lediglich am Ort des operativen Eingriffes eine Öffnung aufweist, ist üblicherweise einstückig ohne jegliche weitere Unterbrechungen ausgebildet. Es stellt somit eine weitgehend sichere und großflächige Abtrennung des sterilen Bereiches vom nichtsterilen Bereich dar, eine Eigenschaft, die sich die erfindungsgemäße Fixiervorrichtung zu Nutze macht. Auf das, auf der Wirkfläche des Haltemittels aufliegende Operationstuch können grundsätzlich beliebig ausgebildete Bedien- oder Fixierelemente, die ihrerseits über ferromagnetische Eigenschaften verfügen, angebracht werden, die je nach Stärke der magnetischen Kräfte am Haltemittel fixiert werden können. So ist es grundsätzlich möglich, eine aus ferromagnetischen Material gefertigte Stativstange auf eine eben ausgebildete Wirkfläche des Haltemittels, getrennt lediglich durch das Abdeckmittel, zu legen, wodurch diese räumlich fixiert wird. An der, dem Operationsbereich zugewandten Ende der Stativstange können an sich bekannte, gelenkförmig ausgebildete Verbindungsmechanismen für das Anbringen weiterer, stativartig ausgebildeter Bedienelemente angelenkt werden, an deren distalen Endbereich wiederum ein optisches Sichtendoskop zur Überwachung eines operativen Eingriffes angebracht werden kann.

Für die im Sterilbereich, das heißt oberhalb des Abdeckmittels angeordneten Bedien- bzw. Fixierelemente gilt, wie für alle anderen im Sterilbereich einzusetzenden Werkzeuge das Erfordernis einer, wenn möglich leichten und schnellen Sterilisierbarkeit. Diesem Erfordernis kann jedoch mit Gelenkmechanismen bekannter Bauart, die jeweils zwei stabförmig ausgebildete Bedienelemente miteinander derart anzukuppeln, sodass sie weitgehend unbeeinträchtigt um nahezu alle Raumachsen relativ zueinander belegt werden können, nur unbefriedigend oder gar nicht nachgekommen werden, zumal ihr Aufbau zu komplex und mit einer Vielzahl von Spalten versehen ist, in deren Zwischenräume eine vollständige Sterilisation nicht möglich ist.

Um dem Erfordernis einer vollständigen Sterilisation zu genügen sehen besonders geeignete Bedienelemente als Gelenkverbindung an einem ihrer Enden eine pfannenförmig konkav ausgebildete Gelenkstruktur vor, die aus einem ferromagnetischen Material gefertigt ist oder zumindest ferromagnetisches Material aufweist. Aus Gründen vollständiger Sterilisierbarkeit ist zu fordern, dass das ferromagnetische Material eine Glühtemperatur (Curie-Temperatur) aufweist, die über 250°C liegt.

Zum Ankuppeln eines zweiten, vorzugsweise stabförmig ausgebildeten Bedienelement weist dieses eine kugelförmige Gegenkontur an einem seiner Enden auf, die in die sphärisch ausgebildeter Pfannenkontur des ersten Bedienelementes passgenau eingelegt werden kann. Je nach Materialwahl und Gelenkformgröße kann zwischen den, auf Grund magnetischer Kräfte zusammenhaltenden Bedienelementen die Größe der Magnetkräfte gezielt eingestellt werden, die zum einen eine dauerhafte Fügung beider Bedienelemente garantieren, zum anderen aber eine, durch den Operateur gewünschte Drehbeweglichkeit von nahezu 360° relativ zueinander ermöglichen. Auf die weitere Ausgestaltung der im Sterilbereich handzuhabenden Bedien- bzw. Fixierelemente wird unter Bezugnahme auf die konkreten Ausführungsbeispiele in Verbindung mit den Figuren verwiesen.

Die vorstehenden Ausführungen bezogen sich auf die einfachste Ausführungsform einer erfindungsgemäß ausgebildeten Fixiervorrichtung, nämlich auf die bloße Abdeckung der magnetischen Wirkfläche des Haltemittels durch das als Operationstuch ausgebildete Abdeckmittel und ein unmittelbar darauf aufgebrachtes Bedienelement, das vorzugsweise in Art einer Stativstange ausgebildet ist.

Bedingt durch den unmittelbaren Kontakt mit dem Abdeckmittel vermag das, mit dem Haltemittel in magnetischer Wirkverbindung tretende Bedienelement das vorzugsweise aus Textilstoff gefertigte Abdeckmittel auf Grund auftretender Relativbewegungen, die durch die Bedienung des Operateurs zur räumlichen Positionierung des am distalen Ende der Stativanordnung angebrachten endochirurgischen Instruments herrührt, mechanisch derart stark zu beanspruchen, sodass ein Verschleiß des Abdeckmittels nicht auszuschließen ist.

Ferner können sich bei den angesprochenen Relativbewegungen zwischen dem Bedienelement und der Wirkfläche des Haltemittel Falten im Abdeckmittel bilden, die zu einer Dejustierung oder zumindest zu einer gehemmten freien Beweglichkeit der Stativanordnung führen können. Zur Vermeidung derartiger Falten hat es sich als vorteilhaft erwiesen, auf das mit dem Abdeckmittel abgedeckten Haltemittel ein zusätzliches Kappenelement aufzusetzen, das zumindest die Wirkfläche des Haltemittels überdeckt und das auf das Haltemittel mit dem unmittelbar darüber befindlichen Abdeckmittel aufsteckbar bzw. aufsetzbar ist.

Das Kappenelement selbst sieht einen Kappendeckel vor, der vorzugsweise aus ferromagnetischem Material besteht und eine Grundplatte aufweist in die eine Nut eingearbeitet ist, die eine, der Außenkontur des Bedienelementes angepasste Innenkontur aufweist. Auf diese Weise kann beispielsweise ein stabförmiges Bedienelement aus Gründen einer verbesserten Fixierung in die Nut der ferromagnetischen Grundplatte des Kappenelementes eingelegt werden, in der sie magnetisch fest fixiert ist. Je nach Magnetkräfte, die beispielsweise unter Verwendung eines Elektromagneten kontinuierlich gesteuert werden können, kann das Bedienelement in Nutlängsachse verschoben werden sowie gemeinsam mit dem gesamten Kappenelement um das, vorzugsweise einen kreisrunden Außenumfang aufweisende Haltemittel gedreht werden. Für einen oberen Abschluss der einseitig offen ausgebildeten Nut innerhalb der Grundplatte ist eine nichtmagnetische Deckelplatte vorgesehen, die auf die Grundplatte aufgebracht ist, selbst jedoch nicht in magnetische Wechselwirkung mit dem von dem Haltemittel herrührenden Magnetfeld tritt, sodass die Magnetfeldlinien vornehmlich ungeschwächt in die Grundplatte und in das, in der Grundplatte eingebrachte Bedienelement eintreten. Auf diese Weise ist sichergestellt, dass der Großteil des magnetischen Flusses zur Fixierung des in der Grundplatte eingefügten Bedienelementes dient und nicht in magnetfeldabschwächender Weise durch nicht, an der Fixierung beteiligten Komponenten der Fixiervorrichtung gerichtet ist.

Um eine kontrollierte Drehbeweglichkeit des Kappenelementes auf die mit dem Abdeckmittel überdeckten Haltemittels zu Gewähr leisten ohne dabei der Gefahr einer Faltenbildung im Abdeckmittel zwischen dem Haltemittel und dem Kappenelement, sind im Bereich des Umfangrandes am Kappenelement stiftartige Fortsätze, vorzugsweise gleichbeabstandet zueinander derart angeordnet, sodass die stiftartigen Fortsätze beim Aufsetzen des Kappenelementes auf das Haltemittel das Haltemittel an seinem periphären Umfangsrand radial umgeben. Durch die beabstandete Anordnung der stiftartigen Fortsätze am Kappenelement können die sich während des Aufsetzens des Kappenelementes auf das tuchartige Abdeckmittel bildenden Falten in den Zwischenräumen der stiftartigen Fortsätze weitgehend frei entfalten. Hierdurch wird das tuchartige Abdeckmittel über die gesamte Wirkfläche des Halteelementes, die vom Kappenelement überdeckt wird, regelrecht faltenfrei gespannt und verbleibt auch faltenfrei selbst bei einer Drehung des Kappenelementes um das Haltemittel. Die stiftartigen Fortsätze sind im Wesentlichen rund, in Art eines Fingers, ausgebildet, sodass sich die Falten des tuchartigen Abdeckmittels nicht zwischen den Fortsätzen und dem Haltemittel verklemmen können, sondern vielmehr zwischen den stiftartigen Fortsätzen freifallend ausbilden können. Da das Kappenelement mit seiner Grundplatte großflächig auf der gesamten Wirkfläche des Halteelementes, getrennt durch das tuchartige Abdeckmittel, aufliegt, wird das Abdeckmittel auch bei Verdrehung des Kappenelementes um das Haltemittel mechanisch nur gering beansprucht, sodass ein Verschleiß des Abdeckmittels weitgehend ausgeschlossen werden kann. Ferner dient das tuchartige Abdeckmittel zugleich auch als eine Art Gleitzwischenlage zwischen Grundplatte des Kappenelementes und der Wirkfläche des Haltemittels, sodass das Kappenelement, je nach herrschenden magnetischen Kräften vom Operateur ohne jegliches Verklemmen verdreht werden kann. Diese Freibeweglichkeit ist insbesondere für die Feinpositionierung einer am distalen Endbereich der stativartig ausgebildeten Bedienelemente angebrachten Bildaufnahmeeinheit unabdingbar.

Die erfindungsgemäße Fixiervorrichtung dient nicht nur der vorstehend beschriebenen, lösbar festen Verbindung eines stativartig ausgebildeten Bedienelementes an einem fest relativ zum oder am Operationstisch angeordneten Haltemittels, sondern kann überdies auch zur Befestigung bzw. Fixierung des Operationstuches beispielsweise am Operationstisch oder in dessen unmittelbarer Umgebung verwendet werden.

Ein Haltemittel, das beispielsweise eine pfannenartig ausgebildete magnetische Wirkfläche aufweist, kann auch als Fixierelement für das Operationstuch dienen, das auf dem Haltemittel aufliegt und das mit einem Fixierelement, das beispielsweise als Kugel ausgebildet ist, die passgenau in die pfannenförmig ausgebildete Wirkfläche des Haltemittels eingreift. Das Haltemittel selbst befindet sich im Nichtsterilbereich und das kugelförmig ausgebildete Fixierelement kann von Seiten des Sterilbereiches auf das Abdeckmittel in das Haltemittel eingreifend aufgesetzt werden. Derartig ausgebildete Fixierelemente können überdies leicht sterilisiert werden und bei Operationen vielfache Verwendung für Fixieraufgaben finden.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnung exemplarisch beschrieben. Es zeigen:
- Figur 1a, b: schematisierte Seiten- und Draufsicht auf ein Haltemittel,
- Figur 1c: schematisierte Querschnittsdarstellung durch ein Kappenelement
- Figur 2: schematisierte Querschnittsdarstellung durch ein in einem Kappenelement eingefügtes Bedienelement,
- Figur 3: Darstellung der Fixiervorrichtung im Einsatz während einer Operation mit tuchartigem Abdeckmittel sowie
- Figur 4: Darstellung der Bewegungsmöglichkeiten der Fixiervorrichtung im operativen Einsatz.

### Wege zur Ausführung der Erfindung, gewerbliche Verwendbarkeit

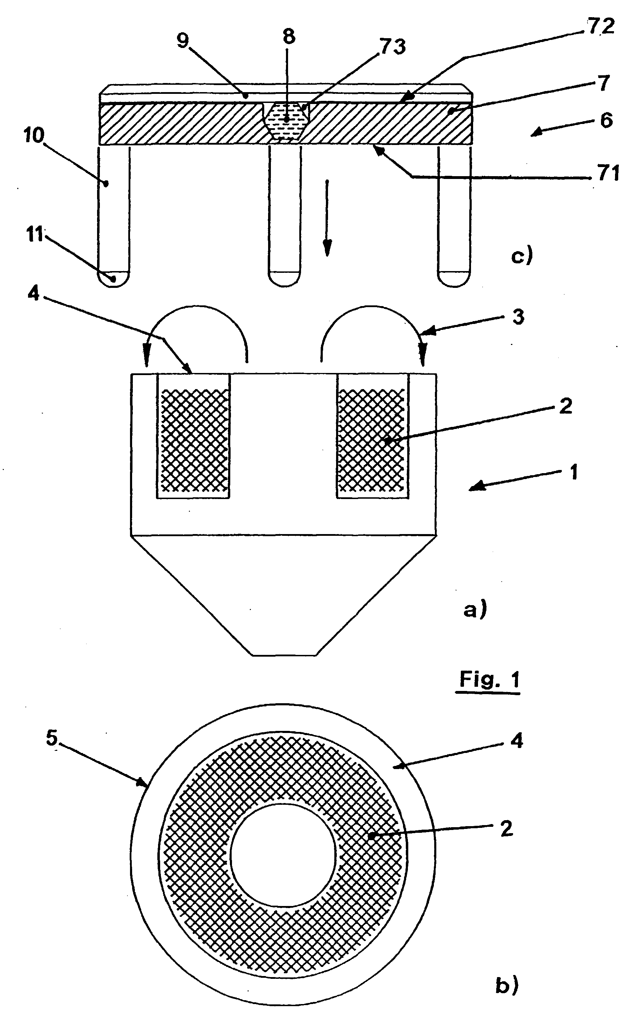

In Figur 1 a ist eine Seitenansicht eines Haltemittels 1 dargestellt, das einen zylinderförmig ausgebildeten Körperabschnitt vorsieht, in den ein ringförmig ausgebildeter Magnet 2 eingearbeitet ist. Grundsätzlich ist es möglich den Magnet 2 als Permanentmagnet oder auch als Elektromagnet auszubilden, wesentlich ist, dass sich das magnetische Feld 3 des Magnetes 2 frei über die Wirkfläche 4 des Haltemittels 1 ausbreiten kann. Der Wirkfläche 4 entgegengesetzt ist am Haltemittel 1 ein kegelförmiger Abschnitt vorgesehen, der fest mit einer nicht dargestellten Stativanordnung verbindbar ist, die zur festen Positionierung des Haltemittels 1 auf dem Boden oder durch entsprechende Anordnung an einer Befestigungsleiste eines Operationstisches fixiert werden kann.

Die Wirkfläche 4 des Haltemittels 1 ist in Figur 1b als eben ausgebildete Fläche in der Draufsicht dargestellt. Der im Querschnitt ringförmige Magnet 2 erzeugt ein im Wesentlichen die Wirkfläche 4 senkrecht nach oben gerichtetes Magnetfeld. Überdies weist das Haltemittel 1 einen kreisrunden Außenumfang 5 auf der, wie im Weiteren noch näher beschrieben wird, in vorteilhafter Weise zur Bedienfreundlichkeit der gesamten Fixiervorrichtung beiträgt.

In Figur 1 c ist in bildliche Gegenüberstellung zu Figur 1 a das Kappenelement 6 in einer Querschnittsansicht dargestellt. Das Kappenelement 6 besteht im Wesentlichen aus einer, die Wirkfläche 4 des Haltemittels 1 seitlich überragenden Grundplatte 7 deren Unterseite 71 in gleicher Weise wie die Wirkfläche 4 eben ausgebildet ist. Die Grundplatte 7 weist überdies an ihrer Oberseite 72 eine einseitig offen ausgebildete Nut 73 auf, deren Innenkontur weitgehend an die Außenkontur eines stabförmig ausgebildeten Bedienelementes 8 angepasst ist. Die gegenseitige Konturanpassung, die im Falle der Ausführungsform gemäß Figur 1 c einer Sechseckform entspricht, dient einem weitgehend innigen und großflächigen körperlichen Kontakt zwischen dem Bedienelement 8 und der Grundplatte 7 was zu einer verbesserten magnetischen Fixierung führt. Ebenso eignet sich ein rechteckiger oder quadratischer Nutquerschnitt mit einer entsprechend diesbezüglichen Querschnittsform des in die Nut 73 einzulegenden Bedienelement 8.

Die Grundplatte 7 ist einheitlich aus ferromagnetischem Material gefertigt, sodass bei entsprechendem Zusammenführen der Grundplatte 7 in Richtung der Wirkfläche 4 des Haltemittels 1 das Magnetfeld 3 weitgehend vollständig in die Grundplatte 7 eindringen kann. Um die Befestigung des Bedienelementes 8 innerhalb der Nut 73 mittels magnetischer Kräfte zu optimieren, ist die Nuttiefe der Nut 73 sehr groß im Vergleich zur Dicke der Grundplatte 7, sodass nur ein möglichst geringer Abstand zwischen Bedienelement 8, das in der Nut eingebracht ist und der Wirkfläche 4 des Halteelementes besteht. Auf diese Weise dringt das Magnetfeld 3 weitgehend ungeschwächt in das aus ferromagnetischem Material gefertigte Bedienelement 8 ein, um dieses zusammen mit der Grundplatte 7 gegen die Wirkfläche 4 des Haltemittels 1 zu fixieren. Es ist jedoch auch möglich die Nut 73 zur Unterseite 71 geöffnet auszuführen.

Zur Verhinderung einer möglichen vertikalen Verkippung des Bedienelementes 8 innerhalb der Nut 73 ist auf der Grundplatte 7 eine Deckelplatte 9 lösbar fest vorgesehen, die ihrerseits aus nicht-ferromagnetischem Material besteht und somit nicht zu einer Magnetfeldschwächung beiträgt.

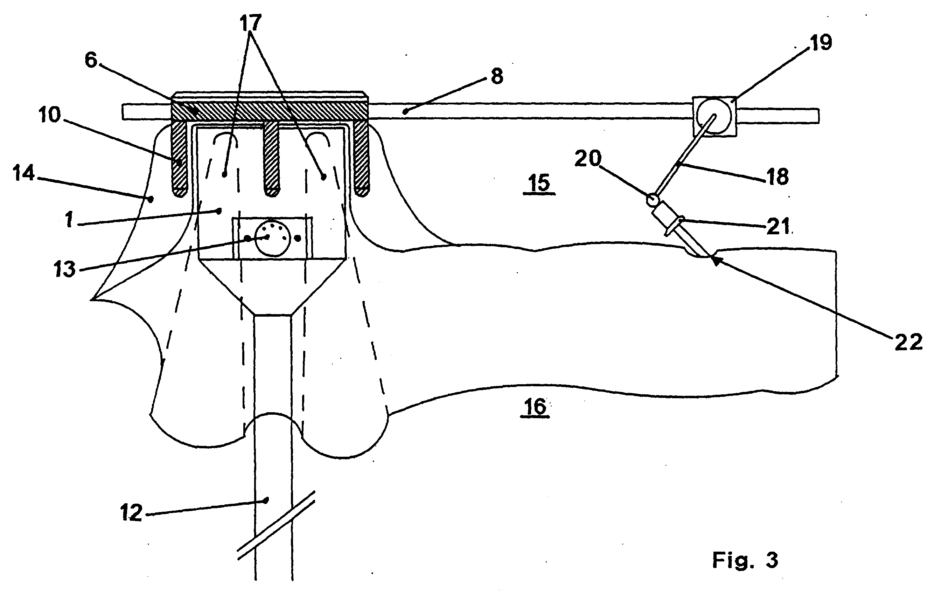

Das Kappenelement 6 weist überdies im Bereich seines ebenfalls kreisrund ausgebildeten Umfangsrand senkrecht von der Grundplatte 7 nach unten stehende, stiftartige Fortsätze 10 auf, die in ein Art runder Finger mit abgerundeten Kuppen 11 ausgebildet sind und nach Aufsetzen des Kappenelementes 6 auf das Haltemittel 1 (siehe Pfeilrichtung) an seinem peripheren Umfangsrand nahezu spielfrei umgeben. Das Kappenelement 6, das auf dem Haltemittel 1 aufsitzt, kann um das Haltemittel 1 gedreht werden, wobei die Anzahl und Anordnung der stiftartigen Fortsätze 10 derart zu wählen sind, dass eine sichere Verdrehung des Kappenelementes 6 relativ zum fest angeordneten Haltemittel 1 ohne Gefahr einer etwaigen Verklemmung bzw. Verkantung möglich ist. Nicht in der Figur 1 ist ein tuchartiges Abdeckmittel vorgesehen, das zwischen dem Kappenelement 6 und der Wirkfläche 4 des Haltemittels 1 eingebracht ist. Hierzu wird im Weiteren auf die Ausführung zu Figur 3 verwiesen.

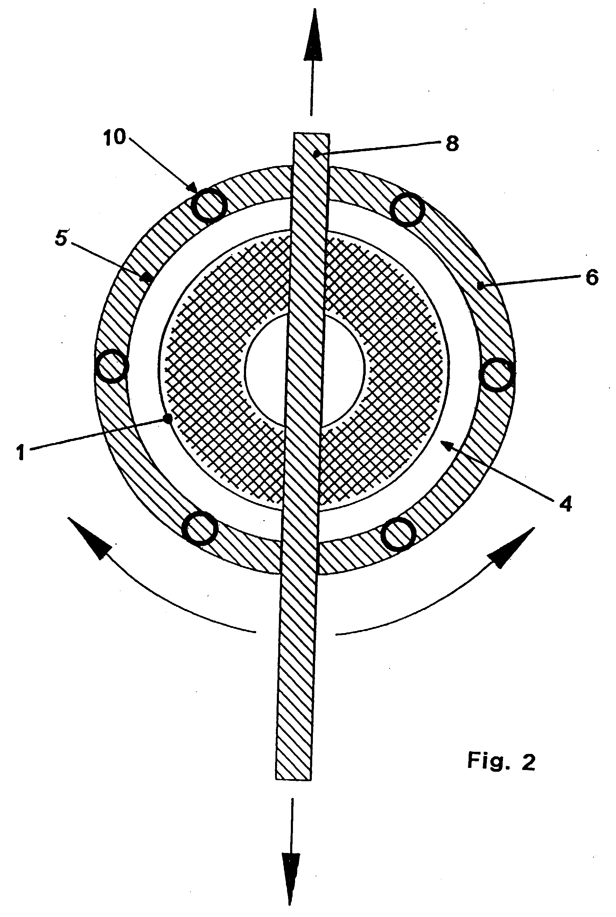

Aus Figur 2, die eine Querschnittsdarstellung des Kappenelementes 6 zeigt, in dem das Bedienelement 8 eingeführt ist und das zusammen mit dem Kappenelement 6 auf der Wirkfläche 4 des Haltemittels 1 aufliegt, ist eine bevorzugte Anordnung der stiftartigen Fortsätze 10 auf dem Kappenelement 6 zu entnehmen. Sechs Fortsätze 10 sind im Bereich des Umfangsrandes des Kappenelementes 6 gleich beabstandet angebracht und schließen weitgehend passgenau den periphären Umfangsrand 5 des Haltemittels 1 in sich ein. Die geometrische Anordnung der Fortsätze 10 am Kappenelement 6 sollte jedoch ein Einbringen eines tuchartigen Abdeckmittels zwischen beiden Komponenten berücksichtigen, sodass ein freies Verdrehen des Kappenelementes 6 relativ zum Haltemittel 1 auch bei einem zwischen beiden Komponenten befindliches Tuch möglich ist.

Durch die magnetischen Kräfte des Magneten 2 werden sowohl das Kappenelement 6 als auch das stabförmig ausgebildete Bedienelement 8, das in das Kappenelement 6 eingeführt ist, relativ zum Haltemittel 1 fixiert. Im bevorzugten Falle der Verwendung eines Elektromagneten kann jedoch die Stärke des magnetischen Feldes variiert werden, sodass zum einen das Bedienelement 8 in seiner Längsrichtung relativ zum Kappenelement 6 beweglich geführt werden kann, zum anderen kann vermittels des Bedienelementes 8 das Kappenelement 6 frei um 360° um das Haltemittel 1 rotieren (siehe hierzu jeweils die Pfeile in Figur 2, die die Bewegungsfreiheitsgrade der einzelnen Komponenten angeben). Ist eine gewünschte Stellung erreicht, so kann der Operateur die Magnetfeldstärke des Elektromagneten erhöhen, wodurch die einzelnen Komponenten der Fixiervorrichtung nahezu unbeweglich fixiert sind. Die Ansteuerung des Elektromagneten erfolgt mit üblichen Regelkreisen, die ihrerseits im Nichtsterilbereich in einem Operationssaal vorgesehen sind.

Figur 3 zeigt in schematisierter Seitendarstellung die vollständige Anordnung einer vorteilhaft ausgebildeten, erfindungsgemäßen Fixiervorrichtung mit dem auf einem Standfuß 12 räumlich festverbundenen Haltemittel 1. Der Standfuß 12 verfügt seinerseits über eine vertikal, hähenverstellbare, nicht dargestellte Hebevorrichtung, sodass die erfindungsgemäße Fixiervorrichtung in eine beliebige vertikale Position verfahren werden kann. Das Haltemittel 1 weist in seiner, in Figur 3 dargestellten Ausführungstorm am zylinderförmig ausgebildeten Körperabschnitt eine elektronische Anschlussmöglichkeit 13 auf, über die beispielsweise die gesteuerte Stromversorgung für einen, im Haltemittel 1 integrierten Elektromagnet erfolgen kann. Unmittelbar auf dem Haltemittel 1 ist das Operationstuch respektive ein tuchartiges Abdeckmittel 14 aufgebracht, das den Sterilbereich 15 von dem unter dem Abdeckmittel 14 vorgesehenen Nichtsterilbereich 16 abtrennt. Vom Operationstuch 14 überdeckt sitzt das Kappenelement 6 auf dem Haltemittel 1 unmittelbar auf. Die sich in den Zwischenräumen, zwischen den stegartigen Fortsätzen 10 bildenden Tuchfalten 17 des Operationstuchs 14 hängen frei nach unten. Die Möglichkeit der Tuchfaltenbildung zwischen den stegartigen Fortsätzen 10 Gewähr leistet eine faltenfreie Ausbildung des Operationstuches 14 unmittelbar zwischen der Wirkfläche 4 des Haltemittels 1 und der Grundplatte 7 des Kappenelementes 6. Selbst ein Verdrehen des Kappenelementes 6 um das feststehende Haltemittel 1 führt nicht zu einem großflächigen Verrutschen des Operationstuches 14 sondern das Operationstuch 14 verbleibt vielmehr bewegungslos zwischen beiden Komponenten.

In das Kappenelement 6 ragt ein stabförmig ausgebildetes, aus ferromagnetischem Material bestehendes Bedienelement 8, das als eine Art Stativstange für ein weiteres, stativartig ausgebildetes Bedienelement 18 dient. Das stativartig ausgebildete Bedienelement 18 ist mit einer Gelenkeinheit 19 weitgehend drehbeweglich mit dem Bedienelement 8 verbunden. Am distalen Ende des stativartig ausgebildeten Bedienelementes 18 ist wiederum über eine weitere Gelenkeinheit 20 ein endochirurgisches Instrument 21 angebracht, das zielgerichtet durch das Operationstuch 14 in eine Körperöffnung 22 hindurchgeführt ist und dort präzise räumlich positioniert ist. Der Operateur, dessen Aufgabe eine präzise räumliche Positionierung des endochirurgischen Instrumentes 21 innerhalb der Körperöffnung 22 ist, steuert vor einer notwendigen Lageänderung der Stativanordnung den Elektromagneten ab, sodass die Fixiervorrichtung die in Figur 2 beschriebenen Bewegungsmöglichkeiten gestattet. Die Gelenkeinheiten 19 und 20 verfügen über bekannte Selbstfixiermechanismen. Ist die neue Position der Stativanordnung und insbesondere des endochirurgischen Instruments 21 erreicht, so wird die Fixiervorrichtung durch entsprechendes Wiedereinschalten des Elektromagneten neu fixiert.

Neben der erwähnten vertikalen Höhenverstellbarkeit des Haltemittels 1 kann das Haltemittel 1 auch um eine horizontal verlaufende Achse geschwenkt werden, sodass die Wirkfläche 4 des Haltemittels 1 eine Neigung relativ zur Horizontalen einnimmt. Ein derartiger Kippmechanismus kann mittels üblicher Rasterverriegelung beispielsweise realisiert werden.

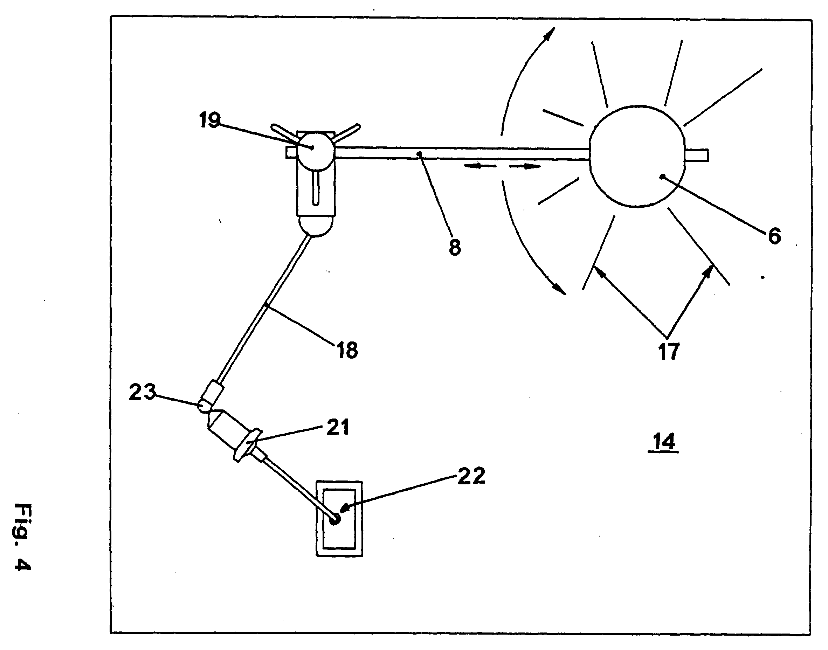

Figur 4 zeigt eine Draufsichtdarstellung der in Figur 3 in Seitenansicht dargestellten Anordnung. Das Bedienelement 8 durchragt das Kappenelement 6, das über das Operationstuch 14, das schematisiert in der Darstellung gemäß Figur 4 den gesamten eingezeichneten rechteckigen Bereich darstellt, auf das nicht sichtbare Haltemittel 1 geschoben ist. Schematisiert sind die Tuchfalten 17 angedeutet.

Die in Figur 4 dargestellten Komponenten befinden sich somit vollständig im sterilen Bereich eines Operationsfeldes. Am distalen Ende des stabförmig ausgebildeten Bedienelementes 8 ist mit einer an sich bekannten Klemmgelenkvorrichtung 19 ein weiteres stativartig ausgebildetes Bedienelement 18 angebracht. An seinem distalen Ende des stativartig ausgebildeten Bedienelementes 18 ist eine pfannenförmig ausgebildete, über magnetische Kräfte verfügende Kontur angebracht, in die eine Kugel 23, die am proximalen Ende des endochirurgischen Instrumentes 21 vorgesehen ist, hineinragt. Die Kugel 23 selbst ist aus ferromagnetischem Material gefertigt und wird durch die Magnetkräfte in der pfannenförmigen Kontur fixiert. Die aus der Magnetkraft herrührende Haltekraft für das endochirurgische Instrument 21 muss dabei so groß sein, dass das Instrument 21 freitragend in einer voreingestellten Position selbsttätig verbleibt. Die Haltekraft darf jedoch nicht so stark sein, dass eine manuelle Bedienung bei entsprechender Lageveränderung unmöglich ist.

Das distale Ende des endochirurgischen Instruments wird durch eine Öffnung im Operationstuch 14 in eine Körperöffnung 22 geführt und dort entsprechend positioniert.

Mit der erfindungsgemäßen Fixiervorrichtung ist es möglich, im Sterilbereich zur unterstützenden Durchführung von Operationen sehr leicht sterilisierbare Komponenten zu verwenden. Mit Hilfe der erfindungsgemäßen Fixiervorrichtung sind keine weiteren Sterilitätsaufwendungen durchzuführen, zumal ein wesentlicher Aspekt der Erfindung die Benutzung des bei jeder Operation ohnehin vorhandenen Operationstuchs ist.

Häufig tritt der Fall auf, dass das Operationstuch am Operationstisch fixiert werden muss. Auch hierzu kann die erfindungsgemäße Fixiervorrichtung dienen, die in der einfachsten Ausführungsform einem Verbindungselement entspricht, das in Figur 4 am distalen Ende des stativartig ausgebildeten Bedienelementes 18 vorgesehen ist. So ist es lediglich erforderlich, als Haltemittel einen Permanentmagneten zu verwenden, der beispielsweise über eine pfannenförmig ausgebildete Wirkfläche verfügt und der im Sterilbereich unterhalb des Operationstuches angeordnet wird. Mit Hilfe einer sehr leicht zu sterilisierenden, aus ferromagnetischem Material gefertigten Kugel, die im Sterilbereich auf das derart ausgebildete Haltemittel aufgesetzt wird, kann das Operationstuch an beliebige Stellen fixiert werden.

### Bezugszeichenliste

- 1: Haltemittel
- 2: Magnet
- 3: Magnetfeld
- 4: Wirkfläche
- 5: runder Umfangsrand
- 6: Kappenelement
- 7: Grundplatte
- 71: Unterseite
- 72: Oberseite
- 73: Nut
- 8: Bedienelement
- 9: Deckelplatte
- 10: stiftartiger Fortsatz
- 11: nicht vergeben
- 12: Standfuß
- 13: elektronische Anschlussmöglichkeit
- 14: tuchartiges Abdeckmittel, Operationstuch
- 15: Sterilbereich
- 16: Nichtsterilbereich
- 17: Tuchfalte
- 18: stativartiges Bedienelement
- 19,20: Gelenkvorrichtung
- 21: endochirurgisches Instrument
- 22: Körperöffnung
- 23: Kugel

## Patentansprüche

1. Fixiervorrichtung für wenigstens ein, im Sterilbereich bei Operationen einsetzbares Bedienelement oder Fixierelement, das räumlich weitgehend frei positionierbar und fixierbar gelagert ist, wobei der Sterilbereich vom Nicht-Sterilbereich im Wesentlichen durch ein tuchartiges Abdeckmittel abgegrenzt ist,
dadurch **gekennzeichnet,** dass ein, im Nicht-Sterilbereich befindliches Haltemittel vorgesehen ist, das wenigstens getrennt durch das Abdeckmittel über eine Wirkfläche mit dem Bedien- oder Fixierelement in Wirkverbindung tritt, und dass die Wirkverbindung auf magnetischen Kräften beruht.

2. Fixiervorrichtung nach Anspruch 1,
dadurch **gekennzeichnet**, dass das Haltemittel als Permanentmagnet ausgebildet ist oder ein Elektromagnet ist, der ein- und aus-schaltbar sowie kontinuierlich regelbar ist.

3. Fixiervorrichtung nach Anspruch 1 oder 2,
dadurch **gekennzeichnet**, dass das Bedien- oder Fixierelement stab-, kugel oder scheibenförmig ausgebildet und ferromganetisches Material aufweist.

4. Fixiervorrichtung nach einem der Ansprüche 1 bis 3,
dadurch **gekennzeichnet**, dass das Bedienelement in Art eines Stativs ausgebildet ist und Befestigungsmittel vorsieht, sodass an dessen distalen Ende endochirurgische Werkzeuge oder bildaufnehmende Einheiten anbringbar sind.

5. Fixiervorrichtung nach einem der Ansprüche 1 bis 4,
dadurch **gekennzeichnet**, dass das Haltemittel ortsfest positionierbar und mittels eines Versteilmechanismus vertikal hähenverstellbar ist.

6. Fixiervorrichtung nach Anspruch 5,
dadurch **gekennzeichnet**, dass die Wirkfläche des Haltemittels aus einer horizontalen Lage in eine geneigte Stellung, vorzugsweise in eine, um 30° zur Horizontalen geneigte Stellung überführbar und fixierbar ist.

7. Fixiervorrichtung nach einem der Ansprüche 1 bis 5,
dadurch **gekennzeichnet**, dass die Wirkfläche des Haltemittels eben oder pfannenförmig mit einer konkav sphärischen Form ausgebildet ist.

8. Fixiervorrichtung nach einem der Ansprüche 1 bis 7,
dadurch **gekennzeichnet**, dass das Haltemittel einen kreisrunden Umfangsrand aufweist.

9. Fixiervorrichtung nach einem der Ansprüche 1 bis 8,
dadurch **gekennzeichnet**, dass ein Kappenelement vorgesehen ist, das zumindest die Wirkfläche des Haltemittels überdeckt und auf das Haltemittel mit dem unmittelbar darüber befindlichen Abdeckmittel aufsetzbar ist

10. Fixiervorrichtung nach Anspruch 9,
dadurch **gekennzeichnet**, dass das Kappenelement einen Kappendeckel vorsieht, der eine ferromagnetische Grundplatte aufweist, in die eine Nut eingearbeitet ist, die eine, der Außenkontur des Bedienelementes angepasste Innenkontur aufweist.

11. Fixiervorrichtung nach Anspruch 10,
dadurch **gekennzeichnet**, dass die Nut einseitig offen in der Grundplatte ausgebildet ist und eine Nuttiefe aufweist, die zumindest der größten Querschnittlänge des Bedienelementes entspricht.

12. Fixiervorrichtung nach einem der Ansprüche 9 bis 11,
dadurch **gekennzeichnet**, dass das Kappenelement eine nicht magnetische Deckelplatte aufweist, die auf der Grundplatte aufgebracht ist.

13. Fixiervorrichtung nach einem der Ansprüche 9 bis 12,
dadurch **gekennzeichnet**, dass am Kappenelement stiftartige Fortsätze angebracht sind, die im Bereich des Umfangrandes des Kappenelementes vorzugsweise gleichbeabstandet zueinander derart angeordnet sind, sodass die stiftartigen Fortsätze beim Aufsetzen des Kappenelementes auf das Haltemittel das Haltemittel an seinem peripheren Umfangsrand umgeben.

14. Fixiervorrichtung nach Anspruch 13,
dadurch **gekennzeichnet**, dass wenigstens zwei, vorzugsweise sechs stiftartige Fortsätze vorgesehen sind.

15. Fixiervorrichtung nach Anspruch 13 oder 14,
dadurch **gekennzeichnet**, dass die stiftartigen Fortsätze eine runde Außenkontur aufweisen.
